# EUROPÄISCHE PATENTSCHRIFT

(11) **EP 0 855 211 B1**
(45) Veröffentlichungstag und Bekanntmachung des Hinweises auf die Patenterteilung: **28.05.2003**
(21) Anmeldenummer: 97117737.3
(22) Anmeldetag: 14.10.1997
(51) Int. Cl.: B01D 63/00, B01D 63/06, B01D 65/00

(54) **Membrantrennvorrichtung**
Membrane separation apparatus
Appareil de séparation à membranes

(30) Priorität: 28.01.1997 DE 19702902
(43) Veröffentlichungstag der Anmeldung: 29.07.1998
(73) Patentinhaber: Uwatech Umwelt- und Wassertechnik GmbH, 29336 Nienhagen (DE)
(72) Erfinder: Kohlheb, Robert, Dr., 29336 Nienhagen (DE); Rautenbach, Robert, Prof. Dr.-Ing., 52056 Aachen (DE)
(74) Vertreter: König, Norbert

(56) Entgegenhaltungen:
- EP-A- 0 448 466
- EP-A- 0 605 826
- DE-A- 2 420 728
- US-A- 4 702 842

## Beschreibung

Die Erfindung betrifft eine Testeinrichtung für Membrantrennverfahren gemäß Oberbegriff des Anspruchs 1.

Es sind aus der EP 0203318 A Testeinrichtungen für Membrantrennverfahren bekannt, mit denen sowohl Filtrations- als auch Permeationsprozesse simuliert werden können. Häufig verwendet man hierzu das Batch-Verfahren, bei dem ein zu prüfendes Medium nach Drosselung des Systemdruckes als Retentat in einem atmosphärischen Gefäß rezirkuliert wird. Der Druckaufbau des Systems wird fast ohne Ausnahme mit Hochdruckpumpen gewährleistet.

Die bekannten Testeinrichtungen für Membrantrennverfahren benutzen Flachmembranen mit kreis- oder viereckförmigen Zuschnitten, die in zweidimensionaler Ebene eingesetzt werden. Diese bekannten Vorrichtungen besitzen Prüfzellen, die für die Querstrom- und Druckfiltration und allgemein für membrantechnische Untersuchungen zur Verfügung stehen. Sie bestehen aus einem zweiteiligen Druckgehäuse, wobei zwischen Gehäuseober- und Gehäuseunterteil eine durch eine Filterunterstützung abgestützte Membran flach eingelagert ist, an die ein zu dieser Membran hin geöffneter Strömungskanal anliegt, dessen Enden mit einem Zulaufstutzen und Konzentratablaufstutzen verbunden ist.

Das Filtrat wird durch einen mit der Membran verbundenen Strömungskanal abgeleitet. Durch den Zulaufstutzen wird das Stoffgemisch senkrecht auf die Membran geleitet, während es über den Konzentratablaufstutzen ebenfalls senkrecht von der Membran weggeleitet wird. Um die Membran vor Abnutzung durch das einströmende Fluid zu schützen, sind Prallbleche zwischen Zulaufstutzen und Membran angebracht. Der an der Membran anliegende Strömungskanal lenkt das Fluid in paralleler spiral- oder mäanderförmiger Bahn über die Membran.

Bei den bekannten Prüfzellen lassen sich die Membranoberflächen für die Stofftrennung nicht ausreichend nutzen. Etwa bis 35 % der Membranoberfläche bleiben ungenutzt. Diese nicht optimale Membranausnutzung wirkt sich nachteilig auf den Durchsatz der Prüfzelle aus. Daher zeigt sich eine sehr zögernde Aufkonzentrierung. Es bedeutet Zeitverlust und ohne Unterdruck-Zirkulation eine unnötige Aufwärmung des Testmediums und unter Umständen eine zusätzlich Kühlung oder Temperierung.

Weiterhin ist bekannt, daß wegen der Mediumbeschaffenheit in überwiegenden Fällen die zu untersuchenden Medien nur beschränkt oder überhaupt nicht transportabel sind. Hierzu einige Gründe:

Das Medium ist als Gefahrenstoff zu betrachten.

Der zu hohe organische Anteil bringt bakterielle Kontaminationen in der Zeit des Transportes und erzeugt damit eine deutliche Änderung der Beschaffenheit.

Das zu untersuchende toxische Medium befindet sich ursprünglich im Ausland: Transport beim Grenzübergang problematisch.

Ein Rücktransport oder eine Beseitigung als Sondermüll des bereits untersuchten Mediums ist zu kostspielig. Die bisherigen Prüfvorrichtungen benötigen sehr oft mehr als 100 1 Volumen für eine membrantechnische Untersuchung.

Zusätzlich wird die Durchführung von Feldversuchen dadurch erschwert, daß die Prüfvorrichtungen nur bedingt mobil sind.

Aus der US 4702842 A ist eine Membrantrennvorrichtung für Umkehrosmose und Ultrafiltration bekannt mit einem Druckbehälter zur Aufnahme von Membranelementen, die von einer zu reinigenden Flüssigkeiten durchströmt werden, und mit einem die Membranelemente umgebenden Zirkulationskanal für die Flüssigkeit.

Die DE 2420728 A betrifft eine Membrantrennvorrichtung zum Abtrennen von Feststoffen aus Flüssigkeiten mit einem Behälter, in dem semipermeable Membraneinheiten parallel angeordnet sind. Hierdurch soll die Gesamtmembranoberfläche vergrößert werden bei gleichzeitiger Verringerung der Größe des Behälters.

Die EP 0448466 A offenbart eine Membrantrennvorrichtung für die Ultra- oder Mikrofiltration von Flüssigkeiten mit Membranen, die aus einem rohrförmigen Bündel von Fasern bestehen, welche mit Rückzirkulation durchströmt werden und in einem Behälter angeordnet sind.

Aus der EP 0605826 A ist eine Filtrationseinrichtung zum Abtrennen von feinen adsorbierenden Partikeln aus Flüssigkeiten bekannt mit einer Vielzahl von Membranen, die senkrecht und parallel in einem Behälter beabstandet zueinander angeordnet sind. Eine Pumpe saugt Prozessflüssigkeit durch die Filtrationseinheit. In die Räume zwischen den Membranen werden Gasbläschen vermittels eines Gebläses eingeblasen.

Die Aufgabe der vorliegenden Erfindung besteht deshalb darin, eine Testeinrichtung für Membrantrennverfahren zu entwickeln, die relativ leicht, tragbar und mobil ist und die schnelle und zuverlässige Untersuchungen umweltrelevanter Stoffe ermöglicht.

Diese Aufgabe wird durch die Erfindung gemäß Anspruch 1 gelöst.

Vorteilhafte und zweckmäßige Weiterbildungen sind in den Unteransprüchen angegeben.

Die erfindungsgemäße Testeinrichtung für Membrantrennverfahren ist als kompaktes und handliches Prüfgerät herstellbar, das keine Druckerhöhungspumpe benötigt. Die Überströmung der Membran wird durch eine unter Druck arbeitende Zirkulationspumpe gewährleistet, die so ausgelegt ist, daß sie auch Flüssigkeiten mit höheren Salzgehalten (bis 15 Gew.-%) pumpt. Die erfindungsgemäße Testeinrichtung für Membrantrennverfahren kann mit relativ kleinen Prüfvolumina, beispielsweise von 100 ml, arbeiten, wodurch eine schnelle Aufkonzentrierung und damit eine kurze Prüfzeit erreichbar ist. Das Totvolumen der Vorrichtung kann sehr klein gehalten werden - unter 1 % -, da sämtliche Räume und Kanäle durchströmt werden. Die Aufwärmung der zu untersuchenden Substanz ist vernachlässigbar, da die Druckunterschiede bei der Zirkulation sehr gering sind - bis maximal 0,5 bar -, da der Arbeitsdruck durch eine externe Druckquelle (beispielweise Gasflasche) eingestellt wird.

Die erfindungsgemäße Testeinrichtung für Membrantrennverfahren eignet sich insbesondere für folgende Membrantrennverfahren:
- Mikrofiltration (Trennung von Mikro-Teilchen bei Suspensionen),
- Ultrafiltration (Separieren von molekularen Anteilen oder von Emulsionen),
- Diafiltration (Rückhaltung von Molekülen und Abführung von salzhaltigen oder alkoholischen Phasen aus wässrigen Lösungen),
- Nanofiltration (molekulare und ionale Fraktionierung),
- Umkehrosmose (Abführung von organischen Lösungen und Aufkonzentrierung von salzhaltigen Gewässern).

Die erfindungsgemäße Testeinrichtung für Membrantrennverfahren ermöglicht einen direkten umweltbezogenen Einsatz für Feldversuche. Dadurch wird die Schnellanalytik für membrantechnische Trennbarkeit der Belastungsstoffe wässriger Lösungen erheblich verbessert.

Die erfindungsgemäße Testeinrichtung für Membrantrennverfahren ist für die Untersuchung unterschiedlich belasteter Wässer geeignet, beispielsweise industrieller Abwasser, Deponie-Sickerwasser, Grundwasser, Abwasser aus Bodenwäsche, Tiefenwasser, Oberflächenwasser, Flußwasser etc. konkrete Beispiele sind:
- Petrochemisches Prozeßwasser: 5 % Chlorpropanol und 1,8 % HCl,
- Technologisches Abwasser aus der chemischen Industrie: 2,4 % Chlorphenol, 5 % NaCl,
- Abwasser aus Bodenwäsche einiger militärischer Objekte: Öl, Reinigungsmittel, Reststoff-Simulation nach ABC-Waffeneinsatz,
- Abwasser aus Ionentauscher-Regenerierungsprozessen: Überwiegend NaCl-Salze mit erhöhter Konzentration,
- Aufbereitung vom Wäschereiabwasser,
- galvanisches Abwasser, Spülbad-Recycling: Schwermetalle und H₂O.

Die Erfindung soll nachfolgend anhand der beigefügten Zeichnung, die ein Ausführungsbeispiel zeigt, näher erläutert werden.

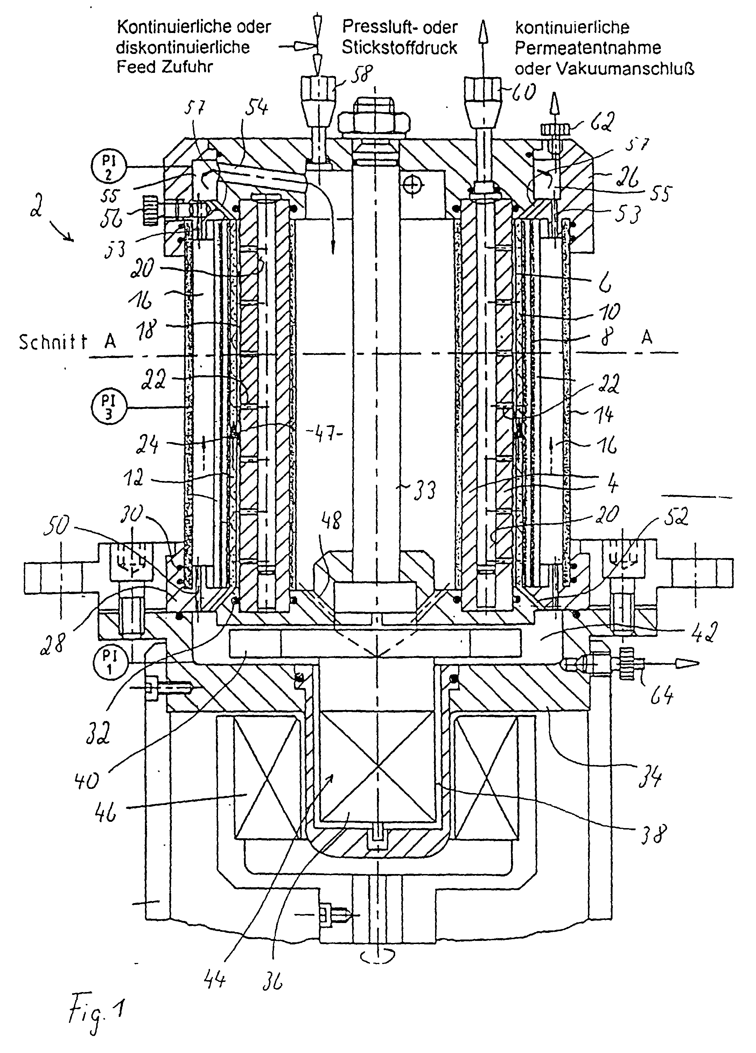

Es zeigt
- Fig. 1: einen senkrechten Schnitt durch eine erfindungsgemäße Testeinrichtung für Membrantrennverfahren und

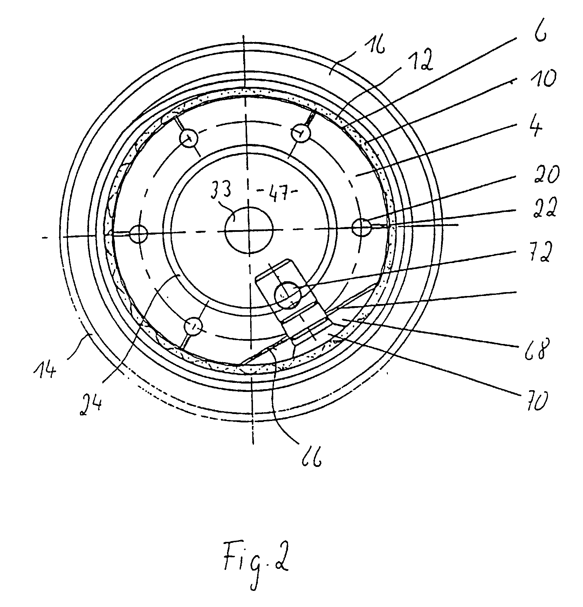

- Fig. 2: einen Horizontalschnitt A-A durch die Testeinrichtung für Membrantrennverfahren nach Fig. 1.

Gleiche Bauteile in den Figuren der Zeichnung sind mit den gleichen Bezugszeichen versehen.

Die Zeichnung zeigt eine Testeinrichtung für Membrantrennverfahren 2 mit einer zylindrischen Membranhalterung 4 - vorzugsweise aus Kunststoff -, auf der eine Membran 6 zylindrisch - also dreidimensional - angeordnet ist, um die herum eine zylindrische Spirale 8 - vorzugsweise aus einer Kunststoffolie - vermittels eines zylindrischen Abstandhalters 10 beabstandet angeordnet ist, wobei der Abstandshalter 10 durch die Spirale 8 auf die Membran 6 aufgespannt wird. Der Abstandshalter 10 ist als Turbulenzeinrichtung im zwischen der Membran 6 und der Spirale 8 gebildeten inneren zylindrischen Ringraum 12 ausgebildet.

Die zylindrische Spirale 8 ist von einem Außenzylinder 14 - vorzugsweise aus Metall - mit Abstand umgeben, so daß zwischen dem Zylinder 14 und der Spirale 8 ein äußerer zylindrischer Ringraum 16 gebildet wird.

Die zylindrische Membranhalterung 4 ist mit den Zylindermantel 18 in axialer Richtung durchsetzenden Sammelbohrungen 20 zu versehen, welche über Querbohrungen 22 im Zylindermantel und über die Membran 6 mit dem Ringraum 6 in Verbindung stehen.

Auf der Zylinderinnenseite der Membranhalterung 4 ist ein zylindrischer Stützkörper 24 - vorzugsweise aus Metall - angeordnet.

Die zylindrische Anordnung aus zylindrischem Stützkörper 24, Membranhalterung 4, Abstandshalter 10, Spirale 8 und Zylinder 14 ist zwischen einer Deckplatte 26 und einer Bodenplatte 28 angeordnet, wobei der äußere Zylinder 14 und die zylindrische Membranhalterung 4 mittels O-Ringen 30, 32 in der Deck- und Bodenplatte abgedichtet angeordnet sind. Die Deck- und Bodenplatte sind miteinander durch einen zentralen Bolzen 33 verspannt.

Unter der Bodenplatte 28 ist ein Pumpengehäuse 34 angeflanscht, in dem sich ein Rotor 36 in einem Rotorraum 38 und ein Pumpenlaufrad 40 in einem Laufradraum 42 einer Zirkulationspumpe 44 befinden, wobei der Rotor 36 vorzugsweise über eine Permanentmagnetkopplung 46 von einem E-Motor (nicht dargestellt) angetrieben wird.

Der von der zylindrischen Membranhalterung 4 bzw. dem zylindrischen Stützkörper 24 eingeschlossene Innenraum 47 steht über wenigstens eine in der Bodenplatte 28 ausgebildete Bohrung 48 saugseitig mit dem Laufradraum 42 in Verbindung, welcher wiederum druckseitig peripher über Bohrungen 50 mit dem äußeren Ringraum 16 und über Bohrungen 52 mit dem inneren Ringraum 12 in Verbindung steht.

Der äußere Ringraum 16 steht über Bohrungen 53, einen in der Deckplatte ausgebildeten Ringkanal 55 und wenigstens einen Zirkulationskanal 54, welcher in der Deckplatte ausgebildet ist, mit dem Innenraum 47 in Verbindung. Die Strömungsgeschwindigkeit im äußeren Ringraum 16 ist mit Hilfe einer Justierschraube 56 einstellbar, mit der der Strömungsquerschnitt der Bohrung 53 einstellbar ist. Der innere Ringraum 12 mit dem Abstandshalter 10 steht über Bohrungen 57 mit dem Ringkanal 55 in Verbindung.

Über die Deckplatte 26 führt ein Einlaßstutzen 58 in den Innenraum 47. Gesteuert über Ventile (nicht dargestellt) strömt über den Einlaßstutzen 58 kontinuierlich oder diskontinuierlich das zu untersuchende Medium, beispielsweise belastetes Wasser. Das Medium wird über den Einlaßstutzen 58 ferner mit Luft oder Stickstoff druckbeaufschlagt.

An die miteinander verbundenen Sammelbohrungen 20 ist ein Auslaßstutzen 60 für kontinuierliche Permeatentnahme und Vakuumanschluß angeschlossen.

Der äußere Ringraum 16 ist über eine Entlüftungseinrichtung 62 entlüftbar.

An den Laufradraum 42 ist druckseitig ein Konzentrat-Entnahmestutzen 64 angeschlossen.

Zur Befestigung der Membran 6 auf der zylindrischen Membranhalterung 4 weist letztere auf der Außenfläche eine Abflachung 66 auf, gegen die eine zylinderabschnittförmige Druckplatte 68 vermittels wenigstens zweier, die Druckplatte 68 und den Mantel der Membranhalterung 4 durchsetzende Spannstifte 70 - vorzugsweise mittels Exzenterbetätigung 72 - preßbar ist, wobei die Membran zwischen der Abflachung 66 und der Druckplatte 68 eingespannt und abgedichtet wird.

Die Funktionsweise der Testeinrichtung für Membrantrennverfahren ist wie folgt:

Eine zu untersuchende belastete (wässrige) Substanz wird über den Eintrittsstutzen 58 in den Innenraum 47 gegeben und über den gleichen Stutzen mit Arbeitsdruck - beispielsweise 3 bis 60 bar, zur Erzielung hoher Ausbeute bis 240 bar - mittels Druckluft oder Inertgasdruck (Stickstoffdruck) beaufschlagt. Die Substanz gelangt über die Bohrungen 48 in die Zirkulationspumpe 44 und wird über die Bohrungen 50 und 52 in den äußeren Ringraum 16 und den inneren Ringraum 12 gefördert, wo die Substanz wieder in den Innenraum 47 über die Bohrungen 53 und 57 und den Zirkulatuionskanal 54 gelangt.

Durch den sich im äußeren Ringraum 16 aufbauenden Druck wird die Spirale 8 gegen den zylindrischen Abstandshalter 10 gedrückt, welcher dadurch die Membran 6 gegen die zylindrische Membranhalterung 4 abdrückt. Die für die Spirale verwendete Kunststoffolie kann eine Stärke von 0,2 bis 2 mm aufweisen und beispielsweise aus PVDF oder PVA bestehen. Die Strömungsquerschnitte sind so gewählt bzw. so einstellbar, daß die Strömungsgeschwindigkeit - 0,0 bis 0,09 m/sec. - im äußeren Ringraum 16 kleiner ist als die Strömungsgeschwindigkeit - 0,5 bis 5 m/sec. - im inneren Ringraum 12 ist. Durch den Arbeitsdruck wird zumindest ein Teil des Permeats durch die Membran gedrückt und gelangt über die Querbohrungen 22 in die Sammelbohrungen 20, aus denen es über den Auslaßstutzen 60 kontinuierlich oder diskontinuierlich abziehbar ist. Die aufkonzentrierte Substanz wird rezirkuliert und kann über den Konzentrat-Entnahmestutzen 64 entnommenen werden. Über den Eintrittsstutzen 58 wird die gleiche Menge Substanz wie die abgeführte Permeatmenge zugeführt.

## Patentansprüche

1. Testeinrichtung für Membrantrennverfahren, mit einer zylindrischen Membran (6), die abgedichtet zwischen einer Deckplatte (26) und einer Bodenplatte (28) angeordnet ist und die beabstandet von einem abgedichtet in der Deckplatte und der Bodenplatte angeordneten zylindrischen Bauteil (14; 8) umgeben ist zur Bildung eines zylindrischen Ringraumes (16),
**gekennzeichnet durch** folgende Merkmale:
a) Eine zylindrische Membranhalterung (4) ist abgedichtet zwischen der Deckplatte (26) und der Bodenplatte (28) angeordnet,
b) die Membran (6) ist auf der zylindrischen Außenfläche der Membranhalterung (4) angeordnet,
c) in der Membranhalterung (4) sind mit der Membran (6) in Verbindung stehende Sammelräume (20) für Permeat ausgebildet, an die ein Permeatauslass (60) angeschlossen ist,
d) die Sammelräume (20) sind **durch** axiale im Mantel der Membranhalterung (4) angeordnete miteinander in Verbindung stehende Bohrungen gebildet,
e) die Bohrungen bzw. Sammelräume (20) stehen über offene in der Außenfläche der Membranhalterung mündende Bohrungen (22) mit der Membran (6) in Verbindung,
f) eine Zirkulationspumpe (44) steht saugseitig mit einem von der zylindrischen Membranhalterung (4) und der Deck- und Bodenplatte eingeschlossenen Innenraum (47) und druckseitig mit dem zwischen Membranhalterung (4) und dem zylindrischen Bauteil (14; 8) gebildeten Ringraum (16) in Verbindung,
g) die Saugseite der Zirkulationspumpe (44) steht über in der Bodenplatte (28) ausgebildete Bohrungen (48) mit dem Innenraum (47) in Verbindung,
h) die Druckseite der Zirkulationspumpe (4) steht über in der Bodenplatte (28) ausgebildete Bohrungen (50) mit dem äußeren Ringraum (16) in Verbindung,
i) der Ringraum (16) ist über einen in der Deckplatte (26) ausgebildeten Zirkulationskanal (54) mit dem Innenraum (47) verbunden,
j) in den Innenraum (47) ist über einen Einlass (58) eine zu untersuchende und mit Druck beaufschlagbare Substanz einspeisbar,
k) die Zirkulationspumpe (44) weist druckseitig einen Auslass (64) zur Entnahme von Konzentrat auf.

2. Testeinrichtung für Membrantrennverfahren nach Anspruch 1, **dadurch gekennzeichnet, dass** die Membran (6) von einem zylindrischen Abstandshalter (10) umgeben ist, welcher von einer zylindrischen Spirale (8) in radialer Richtung belastet ist und die Membran (6) beaufschlagt, wobei zwischen der zylindrischen Spirale (8) und der Membranhalterung (4) bzw. der Membran (6) ein innerer Ringraum (12) gebildet wird, welcher mit der Druckseite der Zirkulationspumpe verbunden ist und mit dem Zirkulationskanal (54) in Verbindung steht.

3. Testeinrichtung für Membrantrennverfahren nach Anspruch 1 oder 2, **dadurch gekennzeichnet, dass** die Druckseite der Zirkulationspumpe (4) über in der Bodenplatte (28) ausgebildete Bohrungen (52) mit dem inneren Ringraum (12) in Verbindung steht.

4. Testeinrichtung für Membrantrennverfahren nach Anspruch 2, **dadurch gekennzeichnet, dass** der Abstandshalter (10) als Turbulenzeinrichtung für die den inneren Ringraum (12) durchströmende Substanz ausgebildet ist.

5. Testeinrichtung für Membrantrennverfahren nach Anspruch 1 oder 2, **dadurch gekennzeichnet, dass** in der Deckplatte (26) ein Ringkanal (55) ausgebildet ist, der über den Zirkulationskanal (54) mit dem Innenraum (47) und über Bohrungen (53, 57) in der Deckplatte mit dem äußeren Ringraum (16) und dem inneren Ringraum (12) verbunden ist.

6. Testeinrichtung für Membrantrennverfahren nach Anspruch 1 oder 3, **dadurch gekennzeichnet, dass** die Zirkulationspumpe (44) von einem Elektromotor über eine Permanent-Magnetkupplung (46) angetrieben ist.

7. Testeinrichtung für Membrantrennverfahren nach Anspruch 5, **dadurch gekennzeichnet, dass** die Strömungsgeschwindigkeit im äußeren Ringraum (16) mittels einer in der Wandung der Bohrung (53) angeordneten Justiereinrichtung (56) einstellbar ist.

8. Testeinrichtung für Membrantrennverfahren nach Anspruch 7, **dadurch gekennzeichnet, dass** die Strömungsgeschwindigkeit im äußeren Ringraum (16) niedriger eingestellt ist als im inneren Ringraum (12).

9. Testeinrichtung für Membrantrennverfahren nach Anspruch 1 oder 2, **dadurch gekennzeichnet, dass** die zylindrische Membranhalterung (4) innenseitig einen zylindrischen Stützkörper (24) aufweist.

10. Testeinrichtung für Membrantrennverfahren nach einem der vorhergehenden Ansprüche, **dadurch gekennzeichnet, dass** die Deckplatte (26) und die Bodenplatte (28) mit Hilfe eines Schraubbolzens (33) miteinander verbunden sind.

11. Testeinrichtung für Membrantrennverfahren nach einem. der vorhergehenden Ansprüche, **dadurch gekennzeichnet, dass** der äußere Ringraum (16) entlüftbar (62) ausgebildet ist.

## Claims

1. Test arrangement for membrane separation methods with a cylindrical membrane (6) which is sealingly located between a cover plate (26) and a base plate (28) and which is surrounded by a spaced cylindrical component (14; 8) which is sealingly located in the cover plate and the base plate in order to form a cylindrical annular space (16)
**characterized by** the following features:
a) A cylindrical membrane holding device (4) is sealingly located between the cover plate (26) and the base plate (28),
b) the membrane (6) is located on the cylindrical outer surface of the membrane holding device (4),
c) collecting spaces (20) for permeate are formed in the membrane holding device (4) which are connected with the membrane (6) and to which a permeate outlet (60) is joined,
d) the collecting spaces (20) are formed by interconnected axial bore holes which are located in the casing of the membrane holding device (4),
e) the bore holes or collecting spaces (20), respectively, are connected with the membrane (6) via open bore holes (22) which lead into the outer surface of the membrane holding device,
f) on its suction side, a circulation pump (44) is connected with an interior space (47) which is confined by the cylindrical membrane holding device (4) and the cover and base plates, and on its delivery side it is connected with the annular space (16) which is formed between the membrane holding device (4) and the cylindrical component (14; 8),
g) the suction side of the circulation pump (44) is connected with the interior space (47) via bore holes (48) which are formed in the base plate,
h) the delivery side of the circulation pump (4) is connected with the outer annular space (16) via bore holes (50) which are formed in the base plate (28),
i) the annular space (16) is connected with the interior space (47) via a circulation channel (54) which is formed in the cover plate (26),
j) a substance which is to be examined can be pressurized and fed into the interior space (47) via an inlet (58),
k) on its delivery side, the circulation pump (44) is provided with an outlet (64) for the sampling of concentrates.

2. Test arrangement for membrane separation methods according to claim 1, **characterized in that** the membrane (6) is surrounded by a cylindrical spacer(10) which is radially loaded by a cylindrical spiral (8) and which pressurizes the membrane (6), whereby an inner annular space (12) is formed between the cylindrical spiral (8) and the membrane holding device (4) or the membrane (6), respectively, which is connected with the delivery side of the circulation pump and interconnected with the circulation channel (54).

3. Test arrangement for membrane separation methods according to claims 1 or 2, **characterized in that** the delivery side of the circulation pump (4) is connected with the inner annular space (12) via bore holes (52) which are formed in the base plate (28).

4. Test arrangement for membrane separation methods according to claim 2, **characterized in that** the spacer (10) forms a turbulence facility for the substance which flows through the inner annulus (12).

5. Test arrangement for membrane separation methods according to claims 1 or 2, **characterized in that** a ring channel (55) is formed in the cover plate (26) which is connected with the interior space (47) via the circulation channel (54) and with the outer annular space (16) and the inner annular space (12) via bore holes (53, 57) in the cover plate.

6. Test arrangement for membrane separation methods according to claims 1 or 3, **characterized in that** the circulation pump (44) is driven by an electric motor through a magneto-electric coupling (46).

7. Test arrangement for membrane separation methods according to claim 5, **characterized in that** the flow rate in the outer annulus (16) can be adjusted by means of an adjusting device (56) which is located in the wall of the bore hole (53).

8. Test arrangement for membrane separation methods according to claim 7, **characterized in that** the flow rate in the outer annular space (16) can be adjusted to be lower than in the inner annular space (12).

9. Test arrangement for membrane separation methods according to claims 1 or 2, **characterized in that** the cylindrical membrane holding device (4) is provided with a cylindrical support body on its inside.

10. Test arrangement for membrane separation methods according to one of the preceding claims, **characterized in that** the cover plate (26) and the base plate (28) are interconnected by means of a bolt (33).

11. Test arrangement for membrane separation methods according to one of the preceding claims, **characterized in that** the outer annular space (16) can be deaerated.

## Revendications

1. Le dispositif d'essai pour un processus de séparation à membranes,
à l'aide d'une membrane cylindrique (6) qui est placée calfeutrée entre une plaque de recouvrement (26) et une plaque de fond (28) et qui par écart est entourée d'un élément cylindrique (14; 8) calfeutré et placé entre la plaque de recouvrement et la plaque de fond pour former un espace annulaire cylindrique (16),
présente les caractéristiques suivantes :
a) un support cylindrique de membrane (4) est placé calfeutré entre la plaque de recouvrement (26) et la plaque de fond (28),
b) la membrane (6) est disposée sur la surface externe cylindrique du support de membrane (4),
c) des espaces collecteurs (20) reliés à la membrane (6) sont constitués dans le support de membrane (4) pour le permeat où une sortie de permeat est raccordée,
d) les espaces collecteurs (20) sont constitués par des perçages axiaux reliés les uns aux autres et disposés dans le manteau du support de membrane (4),
e) les perçages ou les espaces collecteurs (20) sont reliés à la membrane (6) par des perçages ouverts (22) débouchant dans la surface externe du support de membrane,
f) une pompe à circulation (44) est reliée à un espace intérieur renfermé par le support cylindrique de membrane (4) et les plaques de recouvrement et de fond du côté aspiration et, du côté refoulement, à un espace annulaire (16) formé entre le support de membrane (4) et l'élément cylindrique (14; 8),
g) le côté aspiration de la pompe à circulation (44) est relié à l'espace intérieur (47) par des perçages (48) formés dans la plaque de fond (28).
h) le côté refoulement de la pompe à circulation (44) est relié à l'espace annulaire extérieur (16) par des perçages (50) formés dans la plaque de fond (28),
i) l'espace annulaire (16) est relié à l'espace intérieur (47) par un canal de circulation (54) formé dans la plaque de recouvrement (26),
j) une substance à analyser et pouvant être mise en pression peut s'alimenter dans l'espace intérieur (47) par un orifice d'entrée (58),
k) la pompe à circulation (44) montre une sortie côté refoulement (64) pour prélever du concentré.

2. Le dispositif d'essai pour un processus de séparation à membranes d'après 1, se distingue par le fait que la membrane (6) est entourée d'un support d'écartement cylindrique (10) qui est sollicité par une spirale cylindrique (8) en direction axiale et qui presse la membrane (6), alors que se forme entre la spirale cylindrique (8) et le support de membrane (4) ou la membrane (6) elle-même, un espace annulaire intérieur (12) qui est relié au côté refoulement de la pompe à circulation et qui est en liaison avec le canal de circulation (54).

3. Le dispositif d'essai pour un processus de séparation à membranes d'après 1 ou 2, se distingue par le fait que le côté refoulement de la pompe à circulation (4) est relié à l'espace annulaire intérieur (12) par les perçages (52) formés dans la plaque de fond (28).

4. Le dispositif d'essai pour un processus de séparation à membranes d'après 2, se distingue par le fait qu'un support d'écartement (10) est conçu comme dispositif de turbulences pour la substance passant par l'espace annulaire intérieur (12).

5. Le dispositif d'essai pour un processus de séparation à membranes d'après 1 ou 2, se distingue par le fait qu'un canal de ceinture (55) est formé dans la plaque de recouvrement (26) qui est relié à l'espace intérieur (47) ainsi qu'à l'espace annulaire extérieur (16) et l'espace annulaire intérieur (12) par des perçages (53, 57) dans la plaque de recouvrement.

6. Le dispositif d'essai pour un processus de séparation à membranes d'après 1 ou 3, se distingue par le fait que la pompe à circulation (44) est actionnée par un moteur électrique au moyen d'un embrayage magnétique permanent (46).

7. Le dispositif d'essai pour un processus de séparation à membranes d'après 5, se distingue par le fait que la vitesse de débit dans l'espace annulaire extérieur (16) peut se régler à l'aide d'un dispositif d'ajustage (56) fixé dans la paroi du perçage (53).

8. Le dispositif d'essai pour un processus de séparation à membranes d'après 7, se distingue par le fait que la vitesse de débit dans l'espace annulaire extérieur (16) a un réglage inférieur à celle dans l'espace annulaire intérieur (12).

9. Le dispositif d'essai pour un processus de séparation à membranes d'après 1 ou 2, se distingue par le fait que le support cylindrique de la membrane (4) montre un appui cylindrique (24) à l'intérieur.

10. Le dispositif d'essai pour un processus de séparation à membranes d'après l'un des points précités, se distingue par le fait que la plaque de recouvrement (26) et la plaque de fond (28) sont reliées l'une à l'autre par un boulon fileté (33).

11. Le dispositif d'essai pour un processus de séparation à membranes d'après l'un des points précités, se distingue par le fait que l'espace annulaire extérieur (16) a été conçu de manière à pouvoir s'aérer (62).
